# EUROPEAN PATENT APPLICATION

(11) **EP 1 859 695 A2**
(43) Date of publication of application: **28.11.2007**
(21) Application number: 07114670.8
(22) Date of filing: 22.12.2004
(51) Int. Cl.: A41B 9/00, A41D 13/12, A61F 5/24, A61F 5/449

(54) **Support Garment**

(30) Priority: 22.12.2003 DK 200301909
(62) Divisional of application: 04803053.0
(71) Applicant: COLOPLAST A/S, 3050 Humlebaek (DK)
(72) Inventor: Ehmsen, Lena Coloplast A/S Ostomy Division,, 1214KJ Hilversum (NL); Hoejland Olsen, Eskil Coloplast A/S Ostomy Division, 3050 Humlebeak (DK)

(57) **Abstract**

A support garment comprising an elastically extensible body part (30) for exerting an overall pressure on the abdominal region of a patient and a crotch part (31), which parts are interconnected at the front and the back of the garment, the edges of those portions of the two parts which are interconnected at the back of the garment being curved in flat condition and the radii of curvature of opposed parts of the edges being related so that the radius of curvature of the curved edge (33) of the crotch part (31) is smaller than the radius of curvature of the curved edge (32) of the body part (30) provides a support garment for patients with hernia requiring support in the abdominal area which is more comfortable to use and which can be mass produced.

## Description

This invention relates to a support garment, especially a hernia support garment in the form of underpants.

A hernia is a protrusion of an organ or tissue through an abnormal opening in the body. Most hernias occur when a part of intestine slips through a weakness in the abdominal wall, creating a bulge that you can see and feel. Hernias can develop around the navel, in the groin, or any place where you may have had a surgical incision.

In case of hernias with a large opening and a relatively small sac, support belts or bandages may be helpful in holding back the protrusion of selected hernias when surgery is not possible or delayed.

Hernias in connection with stomas also often cause a problem. When a stoma is made at the surface of the abdomen the intestine must pass through the muscles of the abdominal wall, thus a potential site of weakness is immediately created. Stoma hernias may make it difficult to attach a bag properly and sometimes their sheer size is an embarrassment as they can be seen beneath the clothes.

US 4612674 describes underwear having an elongated depression extending on the center line of a rear piece and conforming to the gluteal cleft between the buttocks of the wearer. Accordingly, the underwear, when worn, snugly fits to the curves of the buttocks and the gluteal cleft.

DK patent no. 174536 describes an ostomy support garment in the shape of pants, made out of a compression bandage comprising an abdominal reinforcement, a hole allowing the stoma to pass through the pants as well as a hole in the crotch. The ostomy support garment is custom made for each patient using an extensive measuring and adaptation procedure.

However, the overall pressure of a support garment of this type may create discomfort, especially in the crotch region e.g. when sitting. Furthermore, the hole in the crotch requires the use of a further pair of pants.

It is an object of this invention to provide a support garment for patients with hernia requiring support in the abdominal area which is more comfortable to use than prior art support garments.

A further object of this invention is to provide a support garment for patients with hernia requiring support in the abdominal area and having a crotch part made from a comfortable material.

It is yet another object of this invention to provide a comfortable support garment for patients with hernia requiring support in the abdominal area and which can be mass produced.

### Summary of the invention

The support garment of the invention comprises an elastically extensible body part for exerting an overall pressure on the abdominal region of a patient and a crotch part, which parts are interconnected at the front and the back of the garment, the edges of those portions of the two parts which are interconnected at the back of the garment being curved in flat condition and the radii of curvature of opposed parts of the edges being related so that the radius of curvature of the curved edge of the crotch part is smaller than the radius of curvature of the curved edge of the body part.

It is preferred that the curved edge of the crotch part is essentially convex.

The invention is based on the discovery that by joining the body support part and the crotch part at the back of the garment in the manner defined above, a bulge or pouching is created on the back of the garment and that this bulge or pouching makes more room in the crotch zone on the back of the garment, which consequently becomes more comfortable to use than a garment comprising a closely fitting crotch part. Furthermore, the support garment of the invention may be mass produced reducing the costs as compared to custom made products.

The radii of curvature of the edges may vary along the length of the edges, the variations normally being symmetrically distributed in relation to a vertical centerline of the garment thereby enabling a better fit to the user's anatomy.

In a preferred embodiment of the invention the radius of curvature of the edge on the crotch part is 10-15 mm smaller than the radius of the curvature of the edge on the body part within the parts of the edges showing the largest difference.

The parts of the edges of the body part and the crotch part to be gathered preferably have a circular shape in flat condition.

It should be understood that the edges of the body part and the crotch part may have any desired curved shape in flat condition.

The term 'curved shape' as used herein means that the shape deviates from straight line in a smooth, continuous fashion.

The elastically extensible body part of the invention serves to exert a uniform pressure on the abdominal body region of a patient and thus to suppress a hernia. The pressure exerted should preferably be within the range 15-50 mm Hg (20 - 67 hPa).

The body part is preferably prepared from a knitted material comprising one or more elastic yarns such as elastan yarns. A particularly suitable knitted material comprises between 16 and 60 % and more specifically between 30 and 50% elastan yarn. More preferably it is made from a knitted tubular material so as to avoid uncomfortable seams such as side seams.

The body support part may be provided with a hole for a stoma and the edge of the hole may be stabilized as described in International patent application WO 2004/0069117.

The crotch part of the garment of the invention can be made from any flexible material having a good feel.

The crotch part may have a hole or be open at its lowermost end thus allowing the patient to use a toilet without having to remove the support garment. It may also be provided with a means for closing said hole or opening. Examples of such closing means are Velcro® closure, buttons and corresponding button holes, hooks and eyes, etc.

The interconnection between the body part and the crotch part is preferably in the form of seams, welding, etc., as appropriate depending on the materials

### Brief description of the drawings

The invention will now be described in further detail with reference to the drawings in which,
Figure 1 shows a front view of a preferred embodiment of a support garment according to the invention,
Figure 2 shows a back-side view of the embodiment of a support garment shown in figure 1,
Figure 3 shows schematically an exploded view of another embodiment of a support garment of according to the invention,
Figure 4 shows schematically an exploded view of a body part and a crotch part of a further embodiment of a support garment according to the invention, and
Figure 5 illustrates a vertical cross-sectional view the back of a support garment according to the invention along the line I-I in Figure 2.

### Detailed description

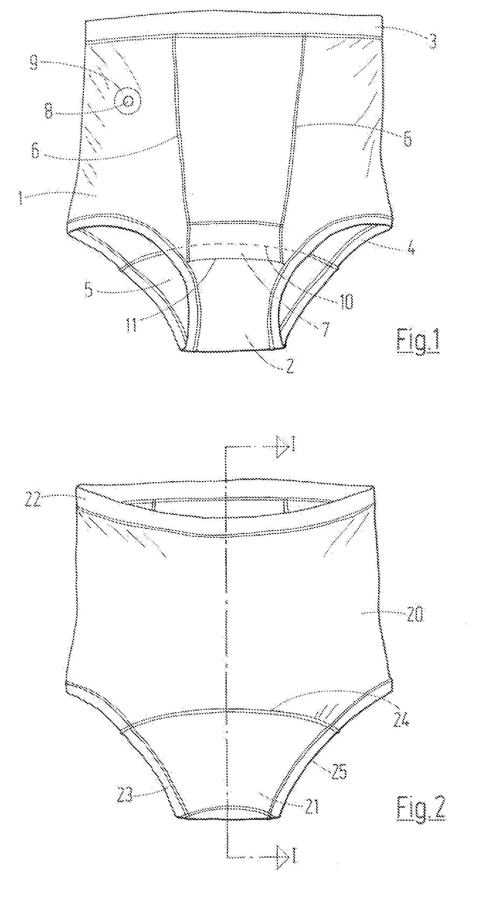

Figure 1 illustrates a front view of a support garment of the invention having a body part 1, a crotch part 2, a waistband 3 attached to the body part 1 and an edging 4 around leg holes 5. The body part 1 has seams 6 for providing an optimum fit to a human body. The crotch part 2 extends from a connection 11 between the body part 1 and the crotch part 2 on the front of the support garment to a connection 10 between the same parts on the back of the support garment. The crotch part 2 is partly covered by a soft material 7, preferably the same material as used in the crotch part 2, and connected in such a way to the body part 1 and the crotch part 2 as to form a passage or fly from the inside of the support garment to the outside of the support garment, in order to make it possible for males to urinate without removing the support garment. The waistband 3 is made out of an elastic material with an inner lining containing anti-slip means, e.g. in the form of a silicon pattern or by use of yarn which has rubber-like qualities, so as to increases the friction to the skin and thus the waistband assists in holding the garment in place. It is also possible to exclude the waistband e.g. by including anti-slip means on the inside of the body part 1. A hole 8 in the body part 1 serves to allow a stoma to pass through the garment. An edging 9 is placed around the hole to stabilise the same. The connection between the body part 1 and the crotch part 2 on the back of the support garment is shown with a broken line 10.

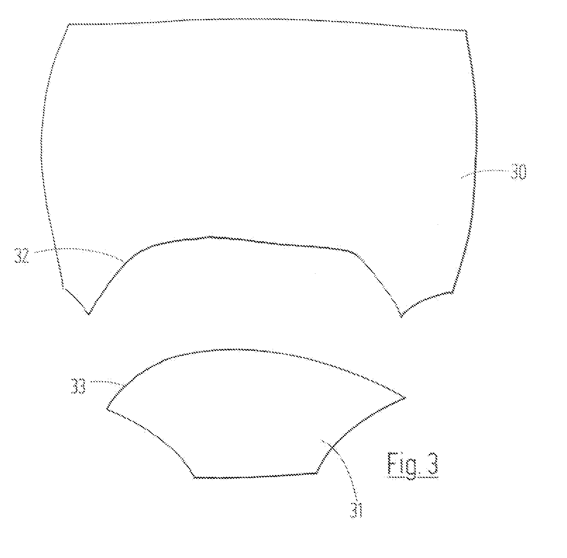

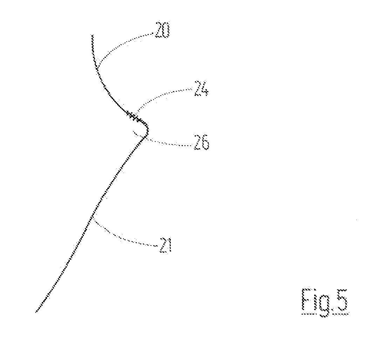

Figure 2 illustrates the back of the support garment of Figure 1, having a body part 20, a crotch part 21 a waistband 22 attached to the body part 20 and an edging 23 around leg holes 25. The body part 20 and the crotch part are sewn together. The edge of the crotch part 21, which is attached to the edge of the body part 20 along a seam 24 is curved, and the radius of curvature of the crotch part 21 is smaller than the radius of curvature of the adjoining curved edge on the body part 20. Hence, the crotch part 21 puffs out as compared to the body part 20, and the bottom of the support garment becomes baggy, as appears from the cross-sectional view shown in Figure 5.

Figure 3 illustrates schematically an exploded view of the back-side of a body part 30 in a flat condition and a crotch part 31 in a flat condition before assembly. The body part 30 comprises a curved edge 33, which is to be connected to a curved edge 32 of the crotch part 31. The radius of the curvature of opposed parts of the edges are related so that the radius of curvature of the crotch part 31 is smaller than the radius of the curvature of the body part 30, and the length of the curved edges on the body part and the crotch part is essentially the same.

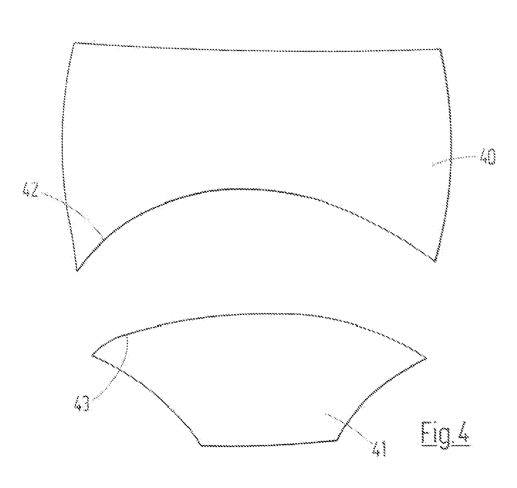

Figure 4 illustrates schematically an exploded view of the back-side of a body part 40 in a flat condition and a crotch part 41 of a further embodiment of the invention in a flat condition before assembly. The body part 40 comprises a curved edge 42, which is to be connected to a curved edge 43 of the crotch part 41. When connected, the connection between the body part 40 and the crotch part 41 is located at a higher level on the support garment compared to the parts shown in figure 3. The curves have a circular shape. The radii of curvature of opposed parts of the edges are related so that the radius of curvature of the crotch part 41 is smaller than the radius of curvature of the body part 41.

In Figure 5, which illustrates a vertical cross-sectional view along the line I-I of the back of the support garment shown in Figure 2a, a body part 20 is attached to a crotch part 21 by means of a seam 24. Other ways of attaching the two parts together are possible as well. As a result of the fact that radius of curvature of the edge of the crotch part 21 is smaller than the radius of curvature of the edge of the body part 20, a pouch or a bagging 26 is formed in the lower part of the support garment.
1. Support garment comprising an elastically extensible body part (1, 20, 30, 40, 50) for exerting an overall pressure on the abdominal region of a patient and a crotch part (2, 21, 31, 41, 51), which parts are interconnected at the front (11) and the back (10, 24) of the garment, the edges of those portions of the two parts which are interconnected at the back of the garment being curved in flat condition and the radii of curvature of opposed parts of the edges being related so that the radius of curvature of the curved edge (33, 43) of the crotch part (2, 21, 31, 41, 51) is smaller than the radius of curvature of the curved edge (32, 42) of the body part (1, 20, 30, 40, 50).
2. Support garment according to embodiment 1 wherein the radius of curvature of the curved edge of the crotch part (2, 21, 31, 41, 51) is 10-15 mm smaller than the radius of curvature of the curved edge of the body part (1, 20, 30, 40, 50).
3. Support garment according to embodiment 1 or 2, wherein the curved edge (33, 43) of the crotch part (2, 21, 31, 41, 51) is convex.
4. Support garment according to embodiment 1 wherein the overall pressure on the abdominal region is 15-50 mmHg (20 - 67 hPa).
5. Support garment according to any one of the preceding embodiments wherein the body part (1, 20, 30, 40, 50) is made from a knitted material.
6. Support garment according to embodiment 5 wherein the body part (1, 20, 30, 40, 50) is made from a knitted tubular material.
7. Support garment according to any one of the preceding embodiments wherein the body part (1, 20, 30, 40, 50) is made out of a material comprising one or more elastic yarns.
8. Support garment according to embodiment 7 wherein the elastic yarn is preferably elastan yarns.
9. Support garment according to embodiment 8 wherein the material of the body part (1, 20, 30, 40, 50) comprises between 16 and 60 % of weight elastan yarn.
10. Support garment according to embodiment 9 wherein the material of the body part (1, 20, 30, 40, 50) comprises between 30 and 50 % of weight elastan yarn.
11. Support garment according to any one of the preceding embodiments wherein the crotch part (2, 21, 31, 41, 51) is made from a material having a good feel.
12. Support garment according to any one of the preceding embodiments further comprising a hole (8) for a stoma.

## Claims

1. Method of joining a body support part and a crotch part at the back of the garment where the body part is elastically extensible and has a curved edge, where the crotch part has a curved edge and where the interconnection between the opposed parts is in the form of a seam such that a pouch or bagging is formed in the lower part of the support garment.

2. Method of joining a body support part and a crotch part at the back of the garment where the body support part is elastically extensible and has a curved edge, where the crotch part has a curved edge and where the interconnection between the opposed parts is in the form of a welding such that a pouch or bagging is formed in the lower part of the support garment.

3. Method of joining a body support part and a crotch part according to claims 1 or 2 where the length of the curved edges is essentially the same.

4. Method of joining a body support part and a crotch part according to claims 1-3 where the connection between the body part and the crotch part is located at a higher level.

5. An elastically extensible body part of a support garment for exerting an overall pressure on the abdominal region of a patient having a curved edge interconnected to a convex edge of an opposing part wherein the length of the curved edges of the two parts is essentially the same and where the radius of curvature of the edges is such that a pouch or a bagging is formed in the lower part of the support garment.

6. A body part interconnected to an opposing part as clamed in claim 5 wherein the opposing part is a crotch part.

7. A body part as claimed in claim 6 wherein the parts of the edges of the body part and the crotch part to be gathered have a circular shape in flat condition.

8. A body part as claimed in claim 7, wherein the circular shape of the edges of the crotch part and the body part are such related that the circle corresponding to the circular shape of the edge of the crotch part has a larger radius than the circle corresponding to the circular shape of the edge of the body part.

9. A crotch part having an essentially convex curved edge which crotch part is interconnected to an elastically extensible body part having a curved edge wherein the length of the curved edges of the two parts is essentially the same and where the radius of curvature of the edges is such that a bulge or pouching is created thereby making more room in the crotch zone.

10. Use of edges on a body part and a crotch part, which have a curved shape in flat condition to create a bulge or pouching on the back of the garment.
